Europäisches Patentamt

European Patent Office

Office européen des brevets

(19)

(11) Publication number: **0 087 238**
**A1**

(12) **EUROPEAN PATENT APPLICATION**

(21) Application number: 83300603.4

(51) Int. Cl.³: **C 12 P 21/02**

(22) Date of filing: 07.02.83

(30) Priority: 08.02.82 GB 8203526
17.11.82 GB 8232809

(43) Date of publication of application:
31.08.83 Bulletin 83/35

(84) Designated Contracting States:
AT BE CH DE FR GB IT LI LU NL SE

(71) Applicant: BIOGEN N.V.
15 Pietermaai
Willemstad Curacao, Netherlands Antilles(NL)

(72) Inventor: Offord, Robin Ewart
11, Chemin Du Calot
CH-1290 Richelieu-sur-Versoix(CH)

(72) Inventor: Keith, Rose
4, Route de Peney
CH-1214 Vernier(CH)

(74) Representative: Bannerman, David Gardner et al,
Withers & Rogers 4 Dyer's Buildings Holborn
London, EC1N 2JT(GB)

(54) Am improved method for preparing human insulin from non-human insulin.

(57) Method for the enzymatic replacement of a C-terminal amino acid in an insulin or insulin-like compound with another amino compound or for the addition of an amino compound to a des-insulin or des-insulin-like compound. In the preferred embodiment the method permits the conversion of swine insulin to human insulin in high yield and in short reaction times by preparing a reaction mixture comprising an insulin or insulin-like compound having a first C-terminal amino acid; an amino compound or salt thereof; a solvent system comprising a buffer, capable of maintaining the pH of the reaction mixture between about 5 and 7, and between about 75% and 97% (vol/vol) of at least one non-aqueous reaction mixture miscible solvent, said reaction mixture miscible solvent including at least about 50% (vol/vol) butane-1, 4-diol, and between about 3% and 25% (vol/vol) water; and at least one protease specific to the cleavage of said first C-terminal amino acid from the insulin or insulin-like compound and at least one protease capable joining to that insulin or insulin-like compound said amino compound or salt thereof, but substantially incapable of cleaving the insulin or insulin-like compound elsewhere under the conditions used; and maintaining the temperature of the mixture between about 4°C and 60°C for about 1 to 2 hours.

DGB/JEA/B22 CIP                     -1-

# AN IMPROVED METHOD FOR PREPARING
# HUMAN INSULIN FROM NON-HUMAN INSULIN

## TECHNICAL FIELD OF INVENTION

This invention relates to an improved method for the enzymatic replacement of a C-terminal amino acid in an insulin or insulin-like compound with another amino compound or to the addition of an amino compound to a des-insulin or insulin-like compound. More particularly, it relates to the enzymatic conversion of the C-terminal alanine of swine insulin to the C-terminal threonine of human insulin. The invention is especially advantaged because it is able to be practiced at sufficiently high yields and at sufficiently short reaction times to permit commercially effective production of human insulin from swine insulin.

## BACKGROUND ART

The use of insulin in the treatment of diabetes mellitus is well known. However, there are insufficient human pancreas glands available to supply the natural human insulin required for these treatments. Accordingly, alternative sources of human insulin have been sought. One possibility might have been the chemical synthesis of human insulin. However, synthesis is expensive and the synthetic approaches to human insulin have been beset by low yields [V. P. Sieber et al., Helv. Chim. Acta, 57, pp. 2617-2621 (1974); V. P. Sieber et al., Helv. Chim. Acta, 60, pp. 27-37 (1977)]. Consequently, non-human insulins have been used as substitutes for human insulin in the treatment of diabetes mellitus and as possible sources of semi-synthetic human insulin.

Non-human insulins have a similar biological activity to human insulin. However, many non-human insulins differ from human insulin in amino acid composition or in amino acid sequence. For example, porcine or swine insulin, one of the more widely used and available non-human insulins, differs from human insulin at the single amino acid located at the C-terminus of the B-chain, i.e., at position B30. In porcine insulin, amino acid B30 is alanine. In human insulin, amino acid B30 is threonine.

The amino acid differences between human insulin and non-human insulins disadvantage the therapeutic effectiveness of substituting non-human insulins for human insulin in the treatment of diabetes because the human body may produce undesirable antibodies in response to the non-human insulin. Plainly, such immunogenic response to the non-human insulins may significantly reduce the effect of the insulin treatment. These amino acid differences may also be related to the pathological changes that occur among diabetic patients maintained on insulin therapy for extended periods of time [M. Ruttenberg, "Human Insulin: Facile Synthesis By Modification Of Porcine Insulin", Science, 177, pp. 623-626 (1972)]. Therefore, it may be more desirable to use a compound identical in structure to human insulin for the treatment of diabetes. Consequently, a method for producing human insulin from non-human insulins in large quantities and in a commercially effective manner is needed.

Two general methods have been developed to effect the enzymatic conversion of non-human insulin into human insulin. These semisynthetic methods are generally characterized by the step of enzymatically removing a portion of the amino acid sequence of a non-human insulin in order to remove at least those amino acids of the non-human insulin that are different from the corresponding amino acids of human insulin. The removed amino acid sequence is then replaced by an amino acid sequence that is identical to that in human insulin. These methods also conventionally entail the steps of protecting the non-human insulin or the added amino acid groups, or both, during cleavage and replacement, reisolating the produced insulin from the reaction mixture, deprotecting it, and purifying the insulin for eventual therapeutic use in the treatment of diabetes.

The first general semisynthetic method is characterized by the removal of an eight amino acid chain from position B30 to position B23 of swine insulin, and its replacement via chemical coupling with a synthetic octapeptide identical to the human (B23-B30)octapeptide. In this method the six carboxy groups of porcine insulin are first protected by reversible blocking. Then, a proteolytic enzyme, for example trypsin, is employed to digest porcine insulin so as to remove amino acids B23-B30. The removed amino acids are replaced by a synthetic octapeptide via peptide coupling to the free carboxyl generated at amino acid B22 as a result of the proteolytic cleavage. After removal of the protective groups and final purification an overall yield of 75% was reported [M. Ruttenberg, "Human Insulin: Facile Synthesis By Modification Of Porcine Insulin", Science, 177, pp. 623-626 (1972)]. However, other human insulin yields reported using this enzymatic cleavage-chemical coupling method have been much lower, [e.g., R. Obermeier and R. Geiger, "A New Semisynthesis Of Human Insulin", Hoppe-Seyler's Z. Physiol. Chem. Bd., 357, pp. 759-767 (1976); R. Obermeier, "Des-Octapeptide-(B23-B30)-Insulin, Starting Material For Human Insulin And Analogues; Studies On Synthesis, Purification And Properties" in Semisynthetic Peptides And Proteins (R.E. Offord and C. Di Bello, eds.) pp. 201-11 (1978)].

In one attempt to avoid the low yields and possible racemization (at B22) that may occur during chemical coupling of the octapeptide to amino acid B22 of the insulin molecule, trypsin was employed to catalyze the coupling of the human (B23-B30)octapeptide to the desoctapeptide-(B23-B30)-swine insulin [K. Inouye et al., "Enzyme-Assisted Semisynthesis of Human Insulin", J. Amer. Chem. Soc., 101, pp. 751-752 (1979); Japanese opened patent application 26881/80]. This enzymatic approach to the coupling reaction improved the insulin yields somewhat (i.e., to about 49%). However, the low yields, long reaction times and complexity of the necessary steps markedly disadvantage this method from the standpoint of the efficient commercial production of insulin. In addition, the method requires the synthesis of a human octapeptide. Although this synthesis is less difficult than the synthesis of the entire insulin molecule, it still disadvantages the method as an effective source of commercial quantities of human insulin.

The second general semisynthetic method of converting swine insulin to human insulin is characterized by the replacement of the single amino acid at the B30 position (alanine in porcine insulin) with threonine (the B30 amino acid of human insulin). Two methods have been developed to accomplish the exchange of the (B30)alanine in porcine insulin with threonine. One of these methods effects the exchange in two steps. First, the (B30)alanine is cleaved from the porcine insulin using a proteolytic enzyme, e.g., carboxypeptidase. The resulting des-alanine (B30)-insulin is then purified and enzymatically coupled to a threonine ester in the presence of organic solvents and an enzyme, like trypsin or achromobacter protease [e.g., K. Morihara et al., "Semi-Synthesis Of Human Insulin By Trypsin-Catalyzed Replacement Of Ala-B30 By Thr In Porcine Insulin", Nature, 280, pp. 412-13 (August 2, 1979) (high concentrations of DMF/EtOH, overall reaction time 28 h, final yield 32%); H. G. Gattner et al., "Enzyme-Catalyzed Semisynthesis With Insulin Derivatives", in Insulin: Chemistry, Structure and Function of Insulin and Related Hormones (D. Brandenburg and A. Wollmer, eds.) pp. 117-23 (1980) (high concentrations of DMF/pyridine acetate, overall reaction time 24 h, final yield 40%); K. Morihara et al., "Achromobacter Protease I-Catalyzed Conversion Of Porcine Insulin Into Human Insulin", Biochemical and Biophysical Research Communication, pp. 396-402 (January 29, 1980) (high concentrations of DMF/EtOH, overall reaction time 110 h, overall yield 52%)]. Plainly, these methods are disadvantaged by the required long reaction times and low yields.

In addition, these processes are disdavantaged because the carboxypeptidase used in them to cleave the (B30)alanine of swine insulin also partially cleaves the (A21)asparagine yielding des-(Ala(B30),Asn(A21))-insulin. This latter insulin displays low biological activity. It also contaminates the resulting product, making purification more difficult [F. H. Carpenter, "Relationship Of Structure To Biological Activity Of Insulin As Revealed By Degradative Studies", Am. Journal of Medicine, 40, pp. 750-58 (1966)]. Moreover, any removal of the des-(Ala(B30),Asn(A21))-insulin from the product mixture, for example, by ion exchange chromatography, adds another step to the process and therefore further disadvantages it in terms of time and material [E.W. Schmitt and H.G. Gattner, "Verbesserte Darstellung von Des-alanyl B30-insulin", Hoppe-Seyler's Z. Physiol. Chem. Bd., 359, pp. 799-802 (1978)].

The second of the B30 exchange methods employs a single step both to remove the (B30)alanine of porcine insulin and to replace it with the desired threonine of human insulin. United States patent 3,276,961 describes one example of this single step exchange. There, porcine insulin is treated with a carboxypeptidase or trypsin in the presence of L-threonine in a water solution at a preferred temperature range of 25°C to 40°C and for a preferred reaction time of 1/2 to 6 h. In the examples recited in that patent (reaction temperature 37°C, reaction time five hours), a three day work-up is said to yield only

- 6 -

360 mg of human insulin from 600 mg of porcine insulin (~ 60% overall yield). Therefore, this method is likewise disadvantaged by low yields and long overall reaction times. In addition, it has been reported that the use of water as a solvent for trypsin digestion permits the trypsin to split the Arg(B22)-Gly(B23) bond thereby contributing to the low yield of insulin and complicating the recovery and purification process, [J. Young and F. Carpenter, "Isolation And Characterization Of Products Formed By The Action Of Trypsin On Insulin", Journal of Biological Chemistry, 236, pp. 743-48 (1961)].

Another example of a single-step exchange at the B30 amino acid of swine insulin to form human insulin is described in Danish patent application 540/81. There, the product insulin is produced from non-human insulin via "transpeptidation" in the presence of a threonine ester. In the process of the Danish application, a reaction mixture comprising insulin, an L-threonine ester of the formula $Thr(R^5)-OR^4$, where $R^5$ is hydrogen or a hydroxyl protective group and $R^4$ is a carboxyl protective group (preferably above about 5:1 mole L-threonine ester/mole of insulin), water (less than 50%, but more than about 10% (vol/vol)), at least one water miscible solvent and trypsin (normally above 1:200, but below about 1:1 (w of trypsin/w of insulin)) is employed. This reaction mixture is reported to enable the conversion of porcine insulin to human insulin under various conditions. For example, the reaction time is stated to vary between several hours and several days depending upon the reaction temperature, the amount of trypsin and the other reaction conditions, and the reaction temperature is stated to range from the freezing point of the reaction solution to 50°C. The following are illustrative of the conditions recited in the Danish application:

Example 1: 18 h, 37°C, 1:10 (w/w) trypsin, ~ 35% $H_2O$

Example 24: 48 h, 12°C, 1:10 (w/w) trypsin, ~ 50% $H_2O$

Example 28: 4 h, 37°C; 1:10 (w/w) trypsin, ~ 30% $H_2O$

The yields of protected insulin (i.e. before deprotection and purification*) recited in that application also depend on the reaction conditions and the particular reaction mixture employed:

Example  1:   60% (18 h, 37°C, 1:10 trypsin, ~ 35% $H_2O$)

Example 24:   97% (48 h, 12°C, 1:10 trypsin, ~ 50% $H_2O$)

Example 28:   46% ( 4 h, 37°C, 1:10 trypsin, ~ 30% $H_2O$)

Therefore, while the latter described method may be an improvement over former methods for converting swine insulin to human insulin, it is still disadvantaged by the long reaction times needed to obtain relatively high yields.  For example, the highest yields of protected human insulin from swine insulin (i.e., without deprotection and purification) reported in Danish application 540/81 required long reaction times:

Example  3:   73%, 18 h

Example  6:   80%, 24 h

Example 10:   80%, 24 h

Example 24:   97%, 48 h

Example 27:   75%, 24 h

Example 34:   93%, 120 h

Long reaction times are commercially disadvantageous considering the material, equipment, and energy factors involved.  In addition, long exposure of a protein to organic solvents may tend to alter the protein's structure and to give rise to changes in its biological or immunological activity.

The process of the Danish application is also disadvantaged by the low yields of protected human insulin produced from swine insulin when shorter reaction times are used:

---

*    Deprotection and purification are most usually effected by inactivating the trypsin (usually with glacial acetic acid), removing it (usually via Sephadex chromatography), deprotecting the insulin (via conventional removal of the protective groups) and purifying the insulin.  These steps may be practiced in about 90% yield.  Accordingly, they lower somewhat the overall yields of any process in which they are required.

Example 28:    46%, 4 h
Example 35:    23%, 4 h
Example 39:    75%, 4 h
Example 41:    46%, 4 h
Example 42:    48%, 4 h

Accordingly, none of the above-described methods has solved the problem of providing high yields of human insulin from non-human sources in a commercially efficient manner.

## DISCLOSURE OF THE INVENTION

This invention solves the problem of obtaining human insulin from non-human sources in high yields and in a commercially efficient manner. In accordance with this invention, we provide a method for the enzymatic replacement of at least one C-terminal amino acid in an insulin or an insulin-like compound with another amino compound or the addition of an amino compound to a des-insulin or insulin-like compound at high yield and in a short reaction time. More specifically, in our preferred embodiment, we provide a method for converting swine insulin into human insulin. This method affords the conversion of swine insulin to human insulin in an overall yield of about 90% after a reaction time of 2 hours or less.

In one embodiment, where a C-terminal amino acid is replaced by another amino group, the method of our invention is characterized by the steps of preparing a reaction mixture comprising an insulin or insulin-like compound having a first C-terminal amino acid; an amino compound or salt thereof; a solvent system comprising a buffer, capable of maintaining the pH of the reaction mixture between about 5 and 7, and between about 75% and 97% (vol/vol) of at least one non-aqueous reaction mixture miscible solvent, said reaction mixture miscible solvent including at least about 50% (vol/vol) butane-1,4-diol, and between about 3% and 25% (vol/vol) water; and at least one protease specific to the cleavage of said first
C-terminal amino acid from the insulin or insulin-like
compound and at least one protease capable of joining to
the resulting insulin or insulin-like compound the said
amino compound or salt thereof, but not cleaving the
insulin or insulin-like compound elsewhere under the conditions used; and maintaining the temperature of the
reaction mixture above about 4°C and below about 60°C for
2 hours or less.

More specifically, in the preferred embodiment
of this invention, the conversion of swine insulin to human
insulin, the method of this invention is characterized by
the steps of preparing a reaction mixture comprising swine
insulin or a swine insulin-like compound; an L-threonine
ester or salt thereof; a solvent system comprising a buffer,
capable of maintaining the pH of the reaction mixture between
about 5 and 7, and between about 75% and 97% (vol/vol) of
at least one non-aqueous reaction mixture miscible solvent,
said reaction mixture miscible solvent including at least
about 50% (vol/vol) butane-1,4-diol, and between about 3%
and 25% (vol/vol) water; and a protease specific to the
desired B29-B30 cleavage and joining reactions; and maintaining the temperature of the reaction mixture above about
4°C and below about 60°C for 2 hours or less.

It should also be understood that the method of
this invention is equally applicable to adding at least
one amino compound to the C-terminus of an insulin or
insulin-like compound, and more preferably to the C-terminus of a des-B30-insulin or other des-insulin.  In that
embodiment, however, only a protease specific to the joining of the desired amino compound or salt thereof to the
C-terminus of the insulin need be employed.  Moreover, in
that embodiment of this invention very short reaction times
are sufficient to attain high yields.

The above-described methods enable the production
of a C-terminal derivative of the desired insulin or insulin-
like compound.  For example, the use of swine insulin and
L-threonine ester with our method produces a B30 ester of human insulin. That ester may then be converted by well-known means to the desired purified insulin or insulin-like compound in high yield.

### BEST MODE OF CARRYING OUT THE INVENTION

In order that the invention herein described may be more fully understood, the following detailed description is set forth.

The "insulins" used in this invention are swine insulin, dog insulin, fin whale insulin, sperm whale insulin, rabbit insulin and primate insulins, from whatever source obtained, including natural, synthetic, semi-synthetic and recombinant DNA sources. The term "insulin-like compounds", as used in describing this invention, is meant to include proinsulins of those insulins and any intermediates between such proinsulins and insulins. These include, for example, split proinsulins, diarginine insulins, desdipeptide proinsulins, and desnonapeptide proinsulins. In the embodiment where we merely add an amino compound to the C-terminus of an insulin or insulin-like compound, as defined above, we may also employ des-insulins or insulin-like compounds particularly des-B30-insulins or insulin-like compounds and des-(B23-B30)-insulins or insulin-like compounds. The method of this invention may also be practiced using a salt or complex of one of the above-described insulins or insulin-like compounds, including a salt or complex with zinc. The insulin or insulin-like compound preferred in the processes of this invention is a swine insulin or insulin-like compound.

The amino compounds useful in the process of our invention include a wide variety of amino compounds such as hydrazine, amino acids, peptides, and other amino compounds that have an amine group available for coupling to the insulin or insulin-like compound in the reaction mixture of this invention. Preferably we employ amino acids.

To assist coupling and to avoid reductions in the yield of the desired insulin derivative, other substituents in the amino compound that may react during our process are usually appropriately protected from such reaction. For example, when we employ an amino acid, we protect the acid functionality with a carboxy protective group and if the amino acid has other reactive substituents, as defined above, we usually also protect them. For example, in the case of threonine, the hydroxy group may be protected with a hydroxy protective group and in the case of lysine, the amine group may be protected with an amine protecting group.

Most preferably, the amino compound employed has the same chirality as the amino acid of the authentic insulin which is to be prepared by the process of this invention, i.e. L-threonine in the preferred conversion of swine insulin to human insulin. Nonetheless, it should be understood that a change in the chirality of the added amino compound, as compared to the authentic insulin, may be advantageous in terms of stability and biological activity. Therefore, compounds where the amino compound has a different chirality than the authentic insulin may be employed in the processes of this invention.

Methods for protecting amino compounds are well known to the art. See, e.g., Wünch: Methoden der Organischen Chemie (Houben Wegl), Vol. XV/1 (E. Müller, ed.) (1974). Protective groups useful in this invention are those that substantially prevent the reaction of the group protected under the reaction conditions of our process and which are removable from the resulting insulin or insulin-like derivative, simply and in a high yield, without affecting the remainder of the insulin molecule after the amino compound is added to the insulin or insulin-like compound by the process of this invention. Methods for removing protective groups are likewise well known to the art. The hydroxyl protecting groups useful in this invention may be any of the hydroxyl protecting groups known to the art that satisfy the above noted limitations. These include, for example, lower alkyl groups of less than 7 carbons and, preferably, less than 5 carbons. The preferred hydroxyl protecting group is tert-butyl. The carboxyl protecting group may likewise be any of the carboxyl protecting groups known to the art that satisfy the above noted limitations. The preferred carboxyl protecting groups are lower alkyl groups like methyl, ethyl, tert-butyl, suitably substituted benzyl groups like diphenylmethyl, and groups of the general formula $CH_3CH_2SO_2R^6$ where $R^6$ is lower alkyl such as methyl, ethyl, propyl or n-butyl, e.g. ethyl methyl sulfone, di-ethyl sulfone, propyl ethyl sulfone, and n-butyl ethyl sulfone. The most preferred carboxyl protecting groups are methyl and tert-butyl. The protected amino acids may also be in the form of their free bases or suitable organic or inorganic salts. In the preferred embodiment of this invention, the amino acid is L-threonine and the carboxy protective group is tert-butyl or methyl, e.g., L-threonine-O-t-butyl ether-t-butyl ester or L-threonine-methyl ester.

In the practice of this invention, a high concentration of the amino compound is preferably employed, and the molar ratio of the amino compound to the insulin or insulin-like compound is also preferably high. For example, the concentration of amino compound in the reaction mixture should be greater than about 100 mM, and the molar ratio of amino compound to insulin or insulin-like compound preferably greater than about 10:1.

The proteolytic enzymes useful in the methods of this invention must have a high specificity for the peptide bond on the carboxyl side of the C-terminal amino acid to be cleaved, usually amino acid B29, under the conditions used. The same enzyme or another one used in combination with the first must also have a high catalytic activity for the formation of a peptide bond between the amino compound and the terminal amino acid of the insulin or insulin-like compound, without at the same time cleaving other parts of the compound. Examples of suitable B29 specific proteolytic enzymes are trypsin, achromobacter proteases and other trypsin-like enzymes such as those described in N. Yoshida et al. "An Anionic Trypsin-Like Enzyme From <u>Streptomyces</u> <u>Eythreus</u>", <u>FEBS</u> Letters, 15, pp. 129-132 (1971) or combinations of those enzymes. The protease should be added to the reaction mixture in an amount such that the weight ratio of protease to insulin is in the range of about 1:1 to 1:100. Preferably, the protease to insulin weight ratio should be about 1:10. It should, of course, be understood that the rate of the reaction may increase as the protease concentration increases. Most usually, proteolytic enzymes are added to the insulin-ester reaction mixture in the presence of water. However, the enzyme need not be added in the presence of water. Instead, it may be added directly to the reaction mixture in solid or liquid form. The enzyme may alternatively be immobilized, for example, on an inert substrate and there contacted with the insulin-containing mixture by contacting that mixture with the immobilized enzyme. Such a process is illustrated in Example 20.

The preferred single protease of this invention is trypsin in any form, provided the B29 specific activity is retained under the conditions used. For example, trypsin may be derived from bovine, porcine or even microbial sources. It may also be in a crystalline or an immobilized form. If a crystalline form is used, it is normally dissolved in water before addition to the reaction mixture, whereas trypsin in the immobilized form is suspended in the reaction mixture. Active trypsin derivatives including acetylated, succinylated and glutaraldehyde-treated trypsins may be used.

The reaction mixture used in this invention is a buffered system. It comprises a buffer, capable of maintaining the pH of the reaction mixture between about 5 and 7. The buffer preferably employed contains Tris or Tris-HCl. However, numerous other well-known compounds that maintain the pH of the reaction mixture within the defined pH range and which do not interfere with the desired insulin reaction or the desired products may be used. Such compounds include suitable inorganic and organic acids and bases, including, for example, hydrochloric acid, formic acid, acetic acid, pyridine, N-methylmorpholine or N-ethylmorpholine. The pH of the reaction mixture of this invention should be maintained between about 5.0 and 7.0, because, if the pH of the mixture is too high, undesirable side reactions that decrease the yield of insulin are more likely to occur. It should, however, be understood that lower pH's favor coupling and higher pH's favor cleavage. Accordingly, the actual pH employed within the range of about 5.0 to 7.0 will depend to some extent on the desired reaction and may represent a balance of effects. Preferably, the pH is monitored during the reaction, but at least it should be checked before incubation. Methods of monitoring pH are well known in the art. For laboratory scale batches one such method is described in Example 1.

The reaction mixture of this invention also includes between about 75% and 97% (vol/vol) of at least one non-aqueous reaction mixture miscible solvent, said reaction mixture miscible solvent including at least about 50% (vol/vol) butane-1,4-diol, and between about 3% and 25% (vol/vol) water. Non-aqueous reaction mixture miscible solvents useful in this invention include, for example, DMSO, glycerol and butane-1,4-diol. Preferably, the reaction mixture of this invention contains about 6% to about 15% (vol/vol) water.

Since the rate of reaction, and hence, the rate of conversion will increase with increasing temperature, the same yields may theoretically be achieved in shorter reaction times if a higher temperature is used. The extent to which the temperature may be increased, however, is limited in that proteases tend to lose their activity at high temperatures. Through the selection of an appropriate solvent system in accordance with this invention, however, protease activity is maintained at temperatures at which in other solvents or solvent systems protease activity would otherwise be impaired. Accordingly, while the temperature of the reaction mixture may be maintained within the range of about 4°C to about 60°C, poorer results in terms of necessary reaction time are observed at low temperatures and poorer results in terms of yield are observed at high temperatures. Accordingly, we prefer to maintain the temperature of the reaction mixture between about 20°C and 42°C.

Depending upon the temperature at which the reaction mixture is maintained, the amount and types of solvent, the amount and type of protease, the amount of amino compound and the particular reaction conditions, yields exceeding 90% may be obtained in reaction times of two hours or less, and if a des-insulin, or more preferably a des-(B30)-insulin, is used, in a reaction time as short as two minutes.

The enzyme-catalyzed reaction of this invention may be stopped by chilling the reaction mixture to a temperature near 0°C and adding glacial acetic acid to inactivate the protease. Methods for stopping the reaction are well known to the art. Likewise, methods for the isolation and deprotection of the resulting insulin-derivative, and purification of the insulin obtained, are well known to the art. For example, the insulin-derivative may be isolated from the other components of the reaction mixture by diluting the reaction mixture with a 1% acetic acid solution, filtering the diluted mixture through a column of Sephadex G-50 or other suitable gel or via anion or cation exchange chromatography, pooling the appropriate portions and freeze drying. Deprotection may be accomplished, for example, by acid using a mixture of trifluoroacetic acid and tryptophan where the carboxy or hydroxy protective group is tert-butyl or a suitably substituted benzyl group. If the carboxy protective group were an alkyl group or an alkyl ethyl sulfone, the deprotection could be accomplished using gentle basic conditions by known methods. Final purification procedures may entail, for example, such steps as filtration, precipitation, centrifugation, washing with ether and drying, and perhaps additional gel filtration steps. It is an advantage of this invention that after these simple steps of deprotection, isolation and purification, e.g., Exmaple 1, are complete, the overall yield of insulin or insulin-like compound produced by the method of this invention is still greater
than 90%.  Of course, should more extensive purification,
for example, to "monocomponent" insulin be desired, the
overall yields will be lower.

In order to describe the present invention so
that it may be more clearly understood, the following
examples of various embodiments are set forth.  These
examples are primarily for the purpose of illustration
and any specific enumeration therein should not be construed as a limitation on the process of this invention.

## Example 1

In the practice of a preferred embodiment of
this invention, we suspended 20 mg of zinc-free porcine
insulin in 270 µl of a buffered solvent system which we
prepared by first dissolving solid Tris-HCl in dimethylsulfoxide (DMSO) to a concentration of 1.5 M.  We then
added two volumes of butane-1,4-diol to one volume of
Tris-HCl/DMSO solution, and finally added a protected
L-threonine (L-threonine-O-t-butyl ether-t-butyl ester).
Since the Tris/DMSO/butane-1,4-diol solution is quite
viscous, we dispensed it by weight rather than by volume.
Accordingly, 660 mg of this solution was added to 62.3 mg
of L-threonine-O-t-butyl ether-t-butyl ester.

We then added to this insulin suspension 36 µl
of a solution of trypsin, prepared by dissolving 3 mg of
a Worthington TPCK inhibited trypsin in 50 µl of water.
The addition of the trypsin and mixing led to the immediate
dissolution of the insulin in the suspension.

It is desirable to check the pH of the reaction
mixture at this stage of the reaction to ensure that the
reaction mixture has not absorbed an excessive amount of
atmospheric moisture.  Accordingly, we placed a 0.5 µl
spot of the reaction mixture on Machery-Nagel 'Duotest'
pH paper (3.5-6.8 range).  The desired reaction mixture
gives an immediate pH indication in the pH 5.9-6.2 range,
and within 15-30 seconds, the same spot should give an indication in the pH 6.5-6.8 range. If the indicator does not respond in this way, the most likely explanation is that the mixture has absorbed water vapor from the atmosphere. If such is the case, then the water concentration in the reaction mixture will be high enough to permit the formation of impurities through hydrolytic reactions of the protease and will result in correspondingly low yields. The reaction mixture of this example produced the appropriate pH response.

After 2 hours at 37°C and stirring, we stopped the reaction by chilling the vessel in a $CO_2$/isopropanol bath and adding 300 µl glacial acetic acid with mixing so as to inactivate the proteolytic and catalytic activity of the trypsin in the reaction mixture. It is essential to inactivate the trypsin at this point because subsequent steps take place in aqueous media, and any trypsin activity remaining after deprotection would reduce yields via undesirable side reactions and cleavage of the insulin product.

We isolated the produced human insulin-Thr(B30)-O-t-butyl ether-t-butyl ester from the other components of the mixture by diluting the resulting solution with 300 µl of acetic acid/water (1:99, by volume) and gel-filtering it through a column of Sephadex G-50 (fine grade; 2.6 cm x 80 cm; acetic acid/water, 1:99 by volume). Losses of product in the column were minimized by previous exposure of the Sephadex to insulin followed by thorough washing. However, such Sephadex conditioning does not need to be repeated for each run. We then pooled the appropriate fractions and freeze-dried them (yield 20.1 mg). This yield corresponds to a yield of 99% relative to the amount of human-insulin-Thr(B30)-O-t-butyl ether-t-butyl ester that would have been produced had all 20 mg of porcine insulin been converted to human insulin-Thr(B30)-O-t-butyl ether-t-butyl ester and isolated.

We deprotected the human insulin-Thr(B30)-O-t-butyl ether-t-butyl ester by dissolving 19.9 mg of the freeze-dried product in 2 ml of redistilled trifluoroacetic acid containing 1 mg tryptophan and maintaining the mixture at 22°C for 45 min. We isolated and purified the resulting human insulin product by reducing the volume of the solution to approximately 0.5 ml by rotary evaporation and then precipitating the insulin with 15 ml of sodium-dried ether. After centrifugation, we washed the precipitate with a further 15 ml of ether, dried it in a vacuum desiccator, and subjected it to gel-filtration as before to yield 18.5 mg. This corresponds to a yield of 93% relative to the theoretical amount of human insulin that would result if all of the human insulin-Thr(B30)-O-t-butyl ether-t-butyl ester produced and recovered in the conversion steps was deprotected, recovered, and purified in the subsequent steps.

Accordingly, the overall yield of human insulin from porcine insulin was 92% relative to the theoretical amount of human insulin that would result if the 20 mg of porcine insulin, which we originally employed, were entirely converted to human insulin and recovered.

## Example 2

We first dissolved 2 mg of des-Ala(B30)-insulin in 10 µl of a 1.5 M solution of Tris-HCl in DMSO. We then added 23 µl of L-threonine-O-t-butyl ether-t-butyl ester to 100 µl of butane-1,4-diol and solid Tris-HCl sufficient to provide an apparent pH of 6.5. We then added to the des-Ala(B30) insulin solution 5 µl of butane-1,4-diol followed by 15 µl of the threonine ester solution and 4 µl of water containing 0.25 mg TPCK-trypsin. The apparent pH was 6.8. Incubation at 37°C for 2 h gave an almost quantitative coupling yield.

## Example 3

We first added two volumes of butane-1,4-diol to one volume of 1.5 M Tris-HCl in DMSO. We then added 223 µl of this mixture to 23 µl of L-Threonine-O-t-butyl ether-t-butyl ester. Next, we added 30 µl of this latter mixture to 2 mg des-Ala(B30)-insulin, followed by the addition of 4 µl of water containing 0.25 mg TPCK-trypsin.

The apparent pH was 6.2. Incubation at 37°C for 1 h gave an almost quantitative coupling yield.

## Example 4

We followed the same method as in Example 3, except that the 4 µl of added water contained 0.5 mg TPCK-trypsin. Incubation at 37°C for 1 h gave an almost quantitative coupling yield.

## Example 5

We first prepared a solution by dissolving 62.3 mg of L-threonine-O-t-butyl ether-t-butyl ester in 660 mg of a mixture of 1.5 M Tris-HCl in DMSO and butane-1,4-diol (1:2 vol/vol). We then added 30 µl of this solution to 2 mg of des-Ala(B30)-insulin. Next, we added 4 µl of water containing 0.25 mg TPCK-trypsin. Incubation at 37°C for 2 min gave about 90% coupling yield.

## Example 6

We treated 20 mg of zinc-free insulin with L-threonine-O-t-butyl ether-t-butyl ester as in Example 1 except that 300 µl of ester solution (not 270 µl) and 40 µl (not 36 µl) of trypsin solution were added. Cellulose acetate electrophoresis displayed an almost quantitative yield after 1 h at 37°C. After 2 h at 37°C followed by the usual work-up we obtained 19.9 mg of human insulin-O-t-butyl ether-t-butyl ester.

## Example 7

We followed the same method as in Example 5 except that zinc-free insulin (not des-Ala(B30)-insulin) was used, and the 4 µl of water contained 0.5 mg TPCK-trypsin. Incubation at 37°C for 10 min gave a 75% coupling yield. Incubation for 40 min gave about 90% coupling yield.

## Example 8

We followed the same method as in Example 5 except that zinc-free insulin (not des-Ala(B30)-insulin) was used and the reaction mixture was incubated at 23°C. After 1 h, the coupling yield was about 90%.

## Example 9

We followed the same method as in Example 8 except that the reaction mixture was incubated at 42°C. After 1 h, the coupling yield was about 90%.

## Example 10

We followed the same method as in Example 8 except that the reaction mixture was incubated at 37°C, and the butane-1,4-diol was replaced by a mixture of glycerol and butane-1,4-diol (1:9 vol/vol). The coupling yield after 1 h was nearly quantitative.

## Example 11

We added 23 µl of L-threonine-0-t-butyl ether-t-butyl ester to 220 µl of a saturated (23°C) solution of Tris hydrochloride in butane-1,4-diol. We added 30 µl of this solution to 2 mg of des-Ala(B30)-insulin followed by the addition of 4 µl of water containing 0.25 mg TPCK-trypsin. The apparent pH was 5.9. Incubation at 37°C for 10 min gave an almost quantitative coupling yield.

## Example 12

We followed the same method as in Example 11 except that 20 µl of the L-threonine ester solution was added to 1.33 mg of zinc-free insulin and to this we added 1.3 µl of water containing 0.16 mg TPCK-trypsin. The apparent pH was 5.6. Incubation at 37°C for 1 h gave almost quantitative coupling yield. This is the most preferred example of the process of our invention.

### Example 13

We dissolved 35 mg L-alanine methyl ester hydrochloride in 100 µl DMSO and 300 µl butane-1,4-diol and we added to this solution solid Tris to an apparent pH of 6.0 to 6.5. We then combined this solution with 10 mg des-(B23-B30)-insulin and added 2.5 mg TPCK-trypsin dissolved in 20 µl water and added another 20 µl water. The apparent pH of the reaction mixture was 6.5. After 2 h at 37°C an 80% coupling yield was observed. After 3 h at that temperature the coupling yield was 90%.

### Example 14

We suspended 5 mg des-(B23-B30)-insulin in 200 µl butane-1,4-diol and added 22.7 mg L-alanine-t-butyl ester hydrochloride dissolved in 20 µl Tris. We then added 1.25 mg TPCK-trypsin dissolved in 10 µl water. The apparent pH was 6.5. We observed coupling yields of 90% after 1 h at 37°C and about 95% after 2 h at that temperature.

### Example 15

We dissolved 72.4 mg $N^\epsilon$Boc-L-lysine-t-butyl ester acetate in 400 µl of a mixture of 1.5 M Tris-HCl in DMSO and butane-1,4-diol(1:2 vol/vol). We then added 20 mg des-Ala(B30)-insulin to 300 µl of that solution and combined it with 40 µl water containing 2.4 mg TPCK-trypsin. The apparent pH was 5.6 to 5.9. After incubation at 37°C for 2 h we obtained about a 100% coupling yield. We recovered 19.5 mg of an L-Lys(B30)-insulin derivative after work-up. This derivative was shown to be pure by cellulose acetate electrophoresis.

### Example 16

We followed the same procedure as in Example 8 except that we incubated the reaction mixture at 4°C. At that temperature we observed a coupling yield of about 60% after 2 h and more than 95% after 24 h.

## Example 17

We prepared 220 μl of a saturated (22°C) solution of Tris-HCl in butane-1,4-diol and added 23 μl L-threonine-O-t-butyl ether-t-butyl ester. We then took 30 μl of that mixture and added it to 2 mg zinc-free insulin. We next added to the mixture 1 μl of a solution of 1.19 mg TPCK-trypsin in 4.8 μl water. The apparent pH was 5.6. We observed coupling yields of less than 10% after 1 h, about 20% after 2 h, 80% after 4 h and greater than 90% after 20 h at 37°C.

## Example 18

We combined 4.19 mg L-threonine methyl ester hydrochloride in 63 μl butane-1,4-diol and adjusted the pH to 5.3-5.6 with solid Tris. We then added 30 μl of that mixture to 1.85 mg zinc-free insulin and added 2 μl of a solution containing 1 mg of TPCK-trypsin in 8 μl water. The apparent pH was 5.6. We observed a yield of protected human insulin of more than 90% after 1 h at 37°C.

## Example 19

We suspended 2 mg zinc-free porcine insulin in 30 μl ester solution, are described in Example 11. We then added 1 μl water and heated the mixture at 37°C for 15 min. After adding 1 μl TPCK-trypsin solution in water (40 mg/ml), we incubated the mixture at 37°C for 2 h with occasional stirring. The coupling yield was almost quantitative.

## Example 20

We rinsed a small column of immobilized trypsin (attached to cross-linked beaded agarose, Sigma Chemical Co.) (7.5 cm x 0.35 cm) with 1% acetic acid and then with a mixture of butane-1,4-diol/water (6:0.8 by vol). We then applied to the column 170 μl of a mixture of a saturated (22°C) solution of Tris HCl in butane-1,4-diol, water and L-threonine-O-t-butyl ether t-butyl ester (223/60/23 by vol). We next added to the column zinc-free insulin dissolved at 37°C in 60 µl of the above-described saturated solution and stopped the column flow. After 2 h at 37°C, we flushed the transformed insulin from the column with a solution of water and butane-1,4-diol (6:0.8 by vol). We collected six 200 µl fractions. We analyzed the fractions by electrophoresis on cellulose acetate. Coupling was almost quantitative.

## Example 21

We prepared a solution of L-threonine-O-t-butyl ether t-butyl ester by adding sequentially to 231 mg of that ester 600 µl butane-1,4-diol, 40 µl acetic acid and 110 µl butane-1,4-diol. We then added 150 µl of this solution to 10 mg zinc-free crude porcine insulin and added 5 µl $H_2O$ to the resulting mixture. After warming the mixture at 37°C for a few minutes, we added 5 µl TPCK-trypsin in water (125 mg/ml) and incubated the mixture at 37°C for 2 h with occasional stirring. During this time the mixture became a completely clear solution. After acidifying the mixture with acetic acid, gel filtering it over Sephadex G50(fine grade) in 1% acetic acid and lyophilizing it, we analyzed the recovered crude ester by electrophoresis on cellulose acetate and by gradient HPLC. The human insulin ether-ester represented about 97% of the product.

While we have hereinbefore presented a number of embodiments of this invention, it should be apparent that our basic construction can be altered to provide other embodiments which utilize the processes of this invention. Therefore, it will be appreciated that the scope of this invention is to be defined by the claims appended hereto rather than the specific embodiments which have been presented hereinbefore by way of example.

CLAIMS:

1. A method for replacing at least one C-terminal amino acid in an insulin or insulin-like compound with another amino group, characterized by the steps of preparing a reaction mixture comprising an insulin or insulin-like compound having a first C-terminal amino acid; an amino compound or salt thereof; a solvent system comprising a buffer, capable of maintaining the pH of the reaction mixture between about 5 and 7, and between about 75% and 97% (vol/vol) of at least one non-aqueous reaction mixture miscible solvent, said reaction mixture miscible solvent including at least about 50% (vol/vol) butane-1, 4-diol, and between about 3% and 25% (vol/vol) water; and at least one protease specific to the cleavage of said first C-terminal amino acid from the insulin or insulin-like compound and at least one protease capable joining to that insulin or insulin-like compound said amino compound or salt thereof, but substantially incapable of cleaving the insulin or insulin-like compound elsewhere under the conditions used; and maintaining the temperature of the mixture between about 4°C and 60°C for about 1 to 2 hours.

2. The method of claim 1, characterized in that the insulin or insulin-like compound is selected from the group consisting of swine insulin, dog insulin, fin whale insulin, sperm whale insulin, rabbit insulin, primate insulins, proinsulins of those insulins, any intermediates between those proinsulins and insulins, and salts or complexes of those insulins or proinsulins, from whatever source obtained.

3. The method of claim 1 or claim 2, characterized in that the insulin is swine insulin, swine proinsulin, any intermediates between swine proinsulin and swine insulin and salts or complexes thereof, from whatever source obtained.

4. The method of any one of claims 1 to 3, characterized in that the non-aqueous reaction mixture miscible solvent is selected from the group consisting of butane-1,4-diol, glycerol and DMSO.

5. The method of any one of claims 1 to 4, characterized in that the solvent system comprises between about 6% and 15% (vol/vol) water.

6. The method of any one of claims 1 to 5, characterized in that the amino compound is selected from the group consisting of hydrazine, amino acids, peptides and other amino compounds having an amine group capable of coupling to the insulin or insulin-like compound in said reaction mixture.

7. The method of any one of claims 1 to 6, characterized in that the amino compound is an amino acid.

8. The method of claim 7, characterized in that the amino acid is L-threonine.

9. The method of claim 7, characterized in that the amino acid is selected from the group consisting of L-threonine-O-t-butyl ether-t-butyl ester, L-threonine-methyl-ester and salts thereof.

10. The method of any one of claims 1 to 9, characterized in that the protease specific to the cleavage of said first C-terminal amino acid and the joining of said amino compound is selected from the group consisting of trypsin, achromobacter proteases and other trypsin-like proteases.

11. The method of claim 10, characterized in that the protease is trypsin in any form that retains the recited specific activity under the conditions used.

12. The method of any one of claims 1 to 11, characterized in that the reaction temperature is between about 20°C and 42°C and the reaction time is between about 1 to 2 hours.

13. A method for replacing the B30 alanine in swine insulin or a swine insulin-like compound with an L-threonine, characterized by the steps of preparing a reaction mixture comprising swine insulin or a swine insulin-like compound; an appropriately protected L-threonine; a solvent system comprising a buffer, capable of maintaining the pH of the reaction mixture between about 5 and 7, and between about 75% and 97% (vol/vol) of at least one non-aqueous reaction mixture miscible solvent, said reaction mixture miscible solvent including at least about 50% (vol/vol) butane-1,4-diol, and between about 3% and 25% (vol/vol) water; and trypsin in any form provided it retains its specific activity for cleaving the B30 alanine from swine insulin or the swine insulin-like compound and joining the L-threonine to that site; and maintaining the temperature of the reaction mixture between about 20°C and 42°C for between about 1 to 2 hours.

14. The method of claim 13 wherein the appropriately protected L-threonine ester is selected from the group consisting of L-threonine-O-t-butyl ether-t-butyl ester, L-threonine-methyl ester and salts thereof.

15. The method of any one of claims 1 to 14 also including the steps of inactivating the protease and isolating, deprotecting and purifying the produced insulin or insulin-like compound.

16. A method for producing human insulin from swine insulin characterized by the steps of preparing a reaction mixture comprising swine insulin or a swine insulin-like compound; an appropriately protected L-threonine; a solvent system comprising a buffer, capable of maintaining the pH of the reaction mixture between about 5 and 7, and between about 75% and 97% (vol/vol) of at least one non-aqueous reaction mixture miscible solvent, said reaction mixture miscible solvent including at least about 50% (vol/vol) butane-1,4-diol, and between about 3% and 25% (vol/vol) water; and trypsin in any form provided it retains its specific activity for cleaving the B30 alanine from swine insulin or the swine insulin-like compound and joining the L-threonine to that insulin or insulin-like compound; maintaining the temperature of the reaction mixture between about 20°C and 42°C for about 1 to 2 hours; inactivating the protease; and isolating, deprotecting and purifying the produced insulin or insulin-like compound.

17. A method for adding at least one amino compound or salt thereof to the C-terminus of a des-insulin or des-insulin-like compound characterized by the steps of preparing a reaction mixture comprising the des-insulin or des-insulin-like compound; the amino compound or salt thereof; a solvent system comprising a buffer, capable of maintaining the pH of the reaction mixture between about 5 and 7, and between about 75% and 97% (vol/vol) of at least one non-aqueous reaction mixture miscible solvent, said reaction mixture miscible solvent including at least about 50% (vol/vol) butane-1,4-diol, and between about 3% and 25% (vol/vol) water; and at least one protease capable of joining said amino compound to the insulin or insulin-like compound, but substantially incapable of cleaving the insulin or insulin-like compound elsewhere under the conditions used; and maintaining the temperature of the reaction mixture between about 4°C and 60°C for about 2 minutes to 1 hour.

18. The method of claim 1, characterized in that said protease is immobilized on an inert substrate.

19. The method of claim 13, characterized in that said trypsin is immobilized on an inert substrate.

20. The method of claim 16, characterized in that said trypsin is immobilized on an inert substrate.

21. The method of claim 17, characterized in that said protease is immobilized on an inert substrate.

## DOCUMENTS CONSIDERED TO BE RELEVANT

| Category | Citation of document with indication, where appropriate, of relevant passages | Relevant to claim | CLASSIFICATION OF THE APPLICATION (Int. Cl. ³) |
|---|---|---|---|
| X | EP-A-0 045 187 (DE FORENEDE BRYGGERIER A/S) <br><br> * Whole document * | 1-5,7-10,12-17 | C 12 P 21/02 |
| X | EP-A-0 017 938 (SHIONOGI) <br><br> * Whole document * | 1-5,7-10,12-17 | |
| X | GB-A-2 069 502 (NOVO) <br><br> * Titlepage; pages 1-3; pages 4,5, example 1; page 8, claims 1,7,9,10; page 9, claim 15 * | 1-5,7-10,12-17 | |
| P,X | CHEMICAL ABSTRACTS, vol. 97, no. 13, 1982, page 481, no. 108524r, Columbus, Ohio, USA & JP - A - 82 79898 (SEIKAGAKU KOGYO CO., LTD.) 19-05-1982 * Abstract * | 1,7-10,12-17 | **TECHNICAL FIELDS SEARCHED (Int. Cl. ³)** <br><br> C 12 P 21/00 |
| P,X | EP-A-0 056 951 (HOECHST) <br><br> * Whole document * <br><br> ---    -/- | 1,7-10,12 | |

The present search report has been drawn up for all claims

| Place of search THE HAGUE | Date of completion of the search 24-05-1983 | Examiner RAJIC M. |
|---|---|---|

European Patent Office

| | DOCUMENTS CONSIDERED TO BE RELEVANT | | | Page 2 |
|---|---|---|---|---|
| Category | Citation of document with indication, where appropriate, of relevant passages | | Relevant to claim | CLASSIFICATION OF THE APPLICATION (Int. Cl. ³) |
| X,Y | EP-A-0 017 485 (DE FORENEDE BRYGGERIER A/S)<br><br>* Titlepage; pages 1-5; pages 21-26; pages 50-51; page 52, claims 11-13 *<br><br>--- | | 1-5,7-10,12-17 | |
| Y | MICROBIOLOGY ABSTRACTS, vol. 15, 1980, Edit. IRL, London, page 15, no. 9050-A15, Oxford, G.B. K. MONHARA et al.: "Achromobacter protease I-catalyzed conversion of poreine insulin into human insulin" &BIOCHEM. BIOPHYS. RES. COMMUN., 92(2), 396-402, 1980<br><br>----- | | 1,7-10,12 | |
| | | | | TECHNICAL FIELDS SEARCHED (Int. Cl. ³) |

The present search report has been drawn up for all claims

| Place of search THE HAGUE | Date of completion of the search 24-05-1983 | Examiner RAJIC M. |
|---|---|---|

EPO Form 1503. 03.82